# EUROPEAN PATENT APPLICATION

(11) **EP 4 729 029 A2**
(43) Date of publication of application: **22.04.2026**
(21) Application number: 25196817.8
(22) Date of filing: 19.08.2025
(51) Int. Cl.: A61F 2/30, A61B 17/72, A61F 2/36

(54) **INTRAMEDULLARY STEM**

(30) Priority: 21.08.2024 US 202463685343 P
(71) Applicant: Howmedica Osteonics Corp., Mahwah, NJ 07430 (US)
(72) Inventor: SCHOLL, Daniel Rudolf, ANDOVER, 07821 (US)
(74) Representative: Regimbeau

(57) **Abstract**

An intramedullary stem includes an elongate body having a length extending from a first end to a second end and a lumen along at least a portion of the length. A first slit extends along the elongate body. The first slit is in between the first and second ends of the elongate body. A fin is attached to the elongate body at the first slit end and extends to a free end remote from the first slit end. The fin is biased in a first position where the free end is external to an outer surface of the elongate body and is movable based on radially inward forces to push the free end toward the elongate body. A second slit extends from the first end of the elongate body to an internal end along the elongate body. Also provided is a method of implanting an intramedullary stem into a bone.

## Description

### CROSS-REFERENCE TO RELATED APPLICATIONS

This application claims the benefit of the filing date of United States Provisional Patent Application No. 63/685,343 filed on August 21, 2024, the disclosure of which is hereby incorporated by reference.

### BACKGROUND

Intramedullary stems are typically used for long bone applications in patients undergoing revision surgeries. Stem implants require stabilization in the medullary canal to prevent failure and loosening of the implant. Some existing intramedullary stems use tapered solid bodies with splines to prevent subsidence in a tibial or femoral canal when it is hollow or lacking good cancellous bone for the stem to attach to. However, such stems are often unable to gain adequate fixation in canals due to the incongruency between the stem shape and the canal shape. Further, with conventional straight cemented or press-fit stems, the distal end of the stem can drive the proximal end of the stem into an unfavorable position.

Some relief of the above deficiencies has been realized when a length of an intramedullary stem is increased. However, while longer intramedullary stem implants may achieve increased fixation within a medullary canal, longer stem implants are disadvantageous in that the stem may be pulled in an anterior direction to be tangent to the curvature of the canal. In longer stems, the angle of the stem relative to the curvature of the canal is driven by a placement of a distal end of the stem within the canal. Such angling of the stem is unfavorable when replicating the position of the distal femur with a final implant. Similarly, when implanted into the tibia, anterior and medial pull or anterior and lateral pull may occur with relatively long stems. Often, this leads to the need for surgeons to utilize offsets in order to prevent the stem location and axis from driving the placement of the stem. Different difficulties are presented with the use of intramedullary stems having a relatively shorter length. While such short stems allow for the stem to flex along a curvature of the medullary canal of the femur, it is difficult to gain adequate fixation in the medullary canal with such a stem.

Accordingly, a need exists for an intramedullary stem that can gain better fixation within a medullary canal.

### BRIEF SUMMARY

The present disclosure addresses the need for an intramedullary stem by providing an intramedullary stem having a hollow body with flexible fins that help the stem gain fixation and be less rigid to help with stress-shielding and active loading of the bone in an inconsistent canal.

In a first aspect, the present disclosure relates to an intramedullary stem for implantation in a bone. The intramedullary stem includes an elongate body having a length extending from a first end to a second end opposite the first end. The elongate body includes a lumen along at least a portion of the length. The stem further includes a first slit extending from a first slit end to a second slit end along the elongate body. The first slit is in between the first and second ends of the elongate body and the first slit has an elongate dimension aligned with the elongate body. The stem further includes a fin attached to the elongate body at the first slit end and the stem extends to a free end remote from the first slit end. The fin is biased in a first position where the free end is external to an outer surface of the elongate body and is movable based on radially inward forces to push the free end toward the elongate body. The stem further includes a second slit extending from the first end of the elongate body to an internal end along the elongate body. The second slit has an elongate dimension aligned with the elongate body.

In some examples, the first slit and the fin are sized such that the free end of the fin is movable into the first slit.

In some examples, a length of the fin between the first slit end and the free end is greater than half of the length of the elongate body.

In some examples, the attachment of the fin to the elongate body is a living hinge.

In some examples, the fin includes a main body and a tip that is angled with respect to the main body, the tip extends from an end of the main body to the free end, and the tip is oriented such that the free end is closer to the elongate body than an opposite end of the tip at the end of the main body.

In some examples, the lumen includes an inner surface and the inner surface is either parallel or tapered relative to a central longitudinal axis of the elongate body.

In some examples, the fin includes one or more ridges on an outward facing surface of the fin.

In some examples, an outward facing surface of the elongate body includes one or more ridges.

In some examples, the elongate body is curved.

In some examples, the fin is a first fin, and the stem further includes a plurality of slits each extending from a first slit end to a second slit end along the elongate body, each slit of the plurality of slits is in between the first and second ends of the elongate body and each slit of the plurality of slits has an elongate dimension aligned with the elongate body. A plurality of fins correspond to the plurality of slits, and each fin of the plurality of fins is attached to a respective slit of the plurality of slits. A circumferential spacing between a first pair of slits from among the first slit and the plurality of slits is different from a second pair of slits from among the first slit and the plurality of slits. At least one slit of the first pair of slits is different from the second pair of slits.

In a second aspect, the present disclosure relates to an implant configured for disposal in a medullary canal of a bone. The implant includes a body having a long dimension sized for disposal along a length direction of the medullary canal of the bone, the body including a hollow core aligned along the long dimension of the body. The implant further includes a fin protruding from the body, the fin is complemented by a slot along the body and the fin is attached to the body at one end of the slot. The fin is configured to flex through the complementary slot and into the hollow core with the application of a radially inward force onto the fin.

In some examples, the fin has a length with a first portion and a second portion extending from the first portion, the first portion is aligned along a first axis and the second portion is aligned along a second axis non-parallel to the first axis.

In some examples, the fin is a first fin and the slot is a first slot, and the implant further includes a second fin complemented by a second slot along the body, the second fin is attached to the body at one end of the second slot, and the second fin is on an opposite side of the body relative to the first fin. In some examples, the implant further includes a third fin and a fourth fin. The third fin is complemented by a third slot along the body and attached to the body at one end of the third slot. The fourth fin is complemented by a fourth slot along the body and attached to the body at one end of the fourth slot. The first, second, third and fourth fins are equally spaced apart. In other examples, the implant further includes a third fin complemented by a third slot along the body and attached to the body at one end of the third slot. A central axis of the third fin is at a first distance from a central axis of each of the first and second fins.

In some examples, the attachment of the fin to the body is a living hinge.

In some examples, the body further includes a slit along the body extending from one end of the body.

In some examples, the hollow core includes an inner surface, the inner surface being either parallel or tapered relative to a central longitudinal axis of the elongate body.

In some examples, the fin includes one or more ridges on an outward facing surface of the fin.

In some examples, an outward facing surface of the body includes one or more ridges.

In some examples, the body is curved.

In a third aspect, the present disclosure relates to a method of implanting an intramedullary stem into a long bone including: inserting an intramedullary stem into a medullary canal of a long bone such that when the intramedullary stem advances into the medullary canal, at least one fin from among a plurality of fins attached to an elongate body of the intramedullary stem bends inward toward a slot in the elongate body aligned with the respective fin, and when the intramedullary stem is advanced to an implantation depth within the medullary canal, at least one fin from among the plurality of fins is biased outward toward a wall of the medullary canal to prevent back out of the intramedullary stem from the medullary canal.

In some examples, when the intramedullary stem is at the implantation depth, the intramedullary stem is offset from a centerline of the medullary canal.

In some examples, during the inserting step, at least one fin from among the plurality of fins is pushed into the slot in the elongate body.

### BRIEF DESCRIPTION OF THE DRAWINGS

A more complete appreciation of the subject matter of the present disclosure and the various advantages thereof may be realized by reference to the following detailed description which refers to the accompanying drawings, in which:
FIG. 1A is a front perspective view of an intramedullary stem accordance with an embodiment of the present disclosure.
FIG. 1B is a back perspective view of the intramedullary stem of FIG. 1A.
FIG. 1C is a cross-sectional view of the intramedullary stem of FIG. 1A taken along line 2-2.
FIG. 2 a side cross-sectional view of the intramedullary stem of FIG. 1A.
FIG. 3 is a side view of the intramedullary stem of FIG. 1A.
FIG. 4A is a perspective view of an intramedullary stem accordance with another embodiment of the present disclosure.
FIG. 4B is a cross-sectional view of the intramedullary stem of FIG. 4A taken along line 2-2.
FIG. 5 is a chart illustrating a method of inserting an intramedullary stem into a medullary canal of a bone according to one embodiment of the present disclosure.
FIG. 6 is a side view of the intramedullary stem of FIG. 1A inserted in a femur.

### DETAILED DESCRIPTION

It should be appreciated that the appended claims or claim elements are not intended to invoke 35 U.S.C. § 112(f) unless the words "means for" or "step for" are explicitly used in the particular claim.

In describing preferred embodiments of the disclosure, reference will be made to directional nomenclature used in describing the human body. It is noted that this nomenclature is used only for convenience and that it is not intended to be limiting with respect to the scope of the present disclosure. As used herein unless stated otherwise, the term "proximal" means closer to the heart and the term "distal" means further from the heart. The term "anterior" means toward the front part of the body, and the term "posterior" means toward the back part of the body. The term "medial" means closer to or toward the midline of the body, and the term "lateral" means further from or away from the midline of the body. The term "inferior" means closer to or toward the feet, and the term "superior" means closer to or toward the crown of the head. In addition, the terms "about," "generally," and "substantially" are intended to mean that slight deviations from absolute are included within the scope of the term so modified.

In one aspect, the present disclosure relates to an intramedullary stem for implantation in a bone. In some examples, the intramedullary stem includes an elongate body having a first slit, a second slit, and a flexible fin extending outward from a surface of an elongate body. The fin can be compressed inward into the elongate body during insertion of the stem into the medullary canal when the fin presses against a wall of the canal.

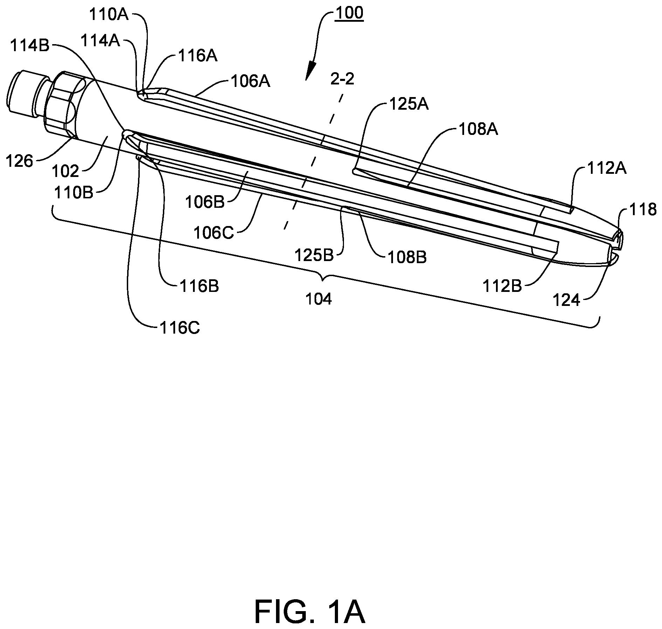

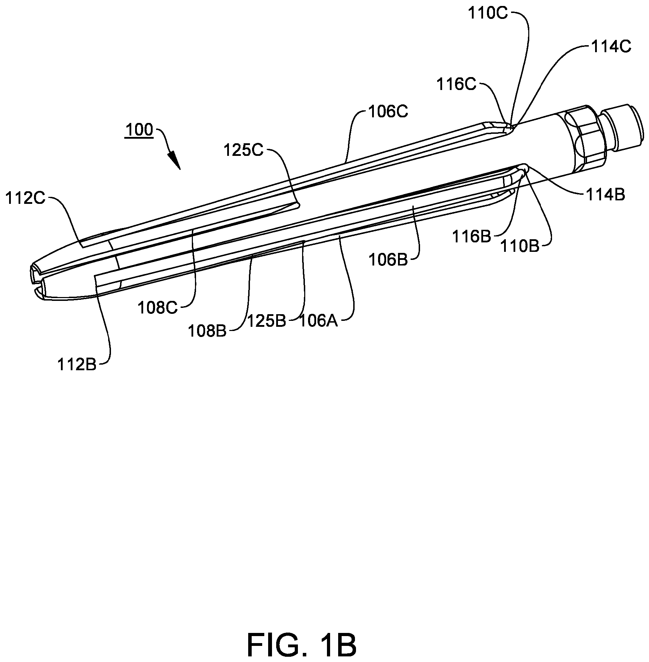

As shown in FIGS. 1A and 1B, an intramedullary stem 100 according to one embodiment of the present disclosure includes an elongate body 102 having a length 104 that extends from a first body end 124 to a second body end 126 opposite first body end. Elongate body 102 has a lumen 118 extending along at least a portion of length 104. A first slit including four first slits, of which first slits 110A-110C are shown, extend from first slit ends 112A-C to second slit ends 114A-C along elongate body 102. First slits 110A-C are in between first and second body ends 124, 126 of elongate body 102 and first slits have an elongate dimension that is aligned with elongate body. In some examples, as shown in FIG. 1A-1B, stem 100 includes four first slits 110A-C and four corresponding fins, of which fins 106A-C are shown. Each first slit 110A-110C may be uniformly spaced around a circumference of stem 100, as is the case in the depicted embodiment. In some examples, stem 100 may have a total of one, two, three or more first slits complemented by the same number of fins. In this manner, intramedullary stem 100 is not limited to the example shown in FIG. 1A-1B. In other examples, the one or more first slits and fins may be non-uniformly spaced around the circumference of the stem.

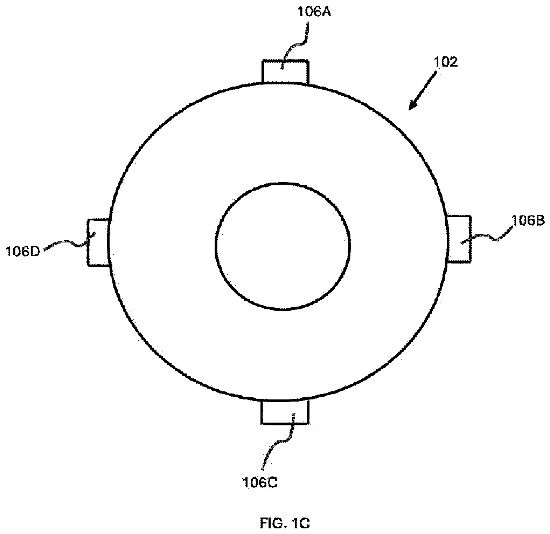

Fins 106A-C are attached to elongate body 102 at respective first slit ends 112A-C and extend to respective free ends 116A-C remote from first slit ends. Fins 106A-C, as depicted, are attached to elongate body 102 via a living hinge and are biased in a first position where free ends 116A-C are external to an outer surface of elongate body 102, as shown in FIGS. 1A-1C and 2-3. Fins 106A-C are movable inward when subject to radially inward forces, such forces pushing one or more of the free ends 116A-C toward elongate body 102. As shown in FIG. 1C, the cross-section along line 2-2 of elongate body 102 of stem 100 includes four fins 106A-D uniformly spaced around a circumference of elongate body. The fins are not limited to those shown in FIGS. 1A-1C and 2-3 and may be of any shape or size. In some examples, the fins may include pores or a porous metal.

Stem 100 further includes four second slits, of which second slits 108A-C are shown, extending from first body end 124 of elongate body 102 to four internal ends, of which internal ends 125A-C are shown, along elongate body. Additionally, second slits 108A-C have an elongate dimension aligned with elongate body 102. Second slits 108A-C allow for flexibility of stem 100 during insertion and removal of stem 100 from the medullary canal. Second slits 108A-C are configured to split an end of stem 100 into flexible sections. Second slits 108A-C allow stem 100 to flex during in vivo loading to prevent pain inside the bone as rigid stem tips can often cause pain during loading. In some examples, the stem may have one, two, three, or more second slits and is not limited to the example shown in FIGS. 1A-1C The size and shape of the second slits is not limited to the example shown in FIGS. 1A-1C, and any size and shape of slit may be used to prevent pain inside the bone from a rigid stem tip.

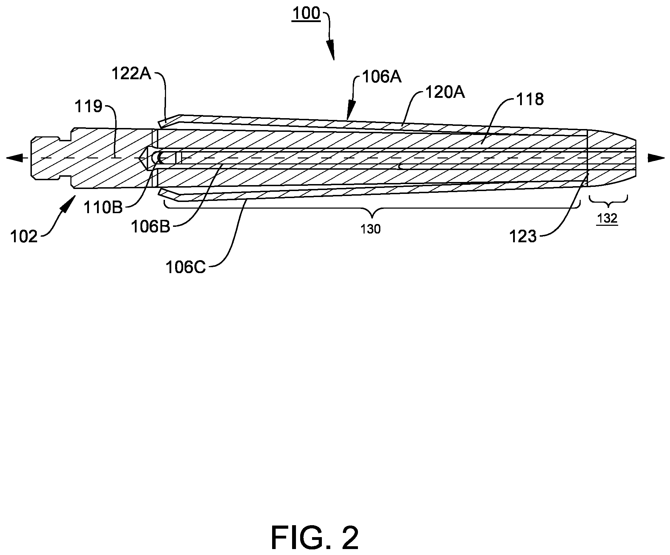

As shown in FIG. 2, lumen 118 of stem 100, in combination with first slits 110A-C, allows respective fins 106A-C to flex inward into lumen 118 of elongate body 102 through first slits 110A-C. In some examples, an inner surface of lumen 118 in an elongate direction may be parallel relative to a central longitudinal axis 119 of elongate body 102. In other examples, the inner surface of lumen 118 may be tapered in the distal direction relative to central longitudinal axis 119 of elongate body 102. A distal end of lumen 118 may include a step 123 for structural support. Step 123 decreases a diameter of lumen 118 so that a first portion 130 of lumen extending along a length of first slits 110A-C has a greater diameter than a second portion 132 of lumen adjacent first body end 124. In some examples, lumen 118 is a single volume that is not separated by any material, and first slits 110A-C are in communication with such lumen. In other examples, first slits 110A-C may be recesses on the elongate body of the stem and lumen 118 may be separated from first slits by a wall. In further variations of these examples, intramedullary stem 100 may have a solid interior with no central lumen.

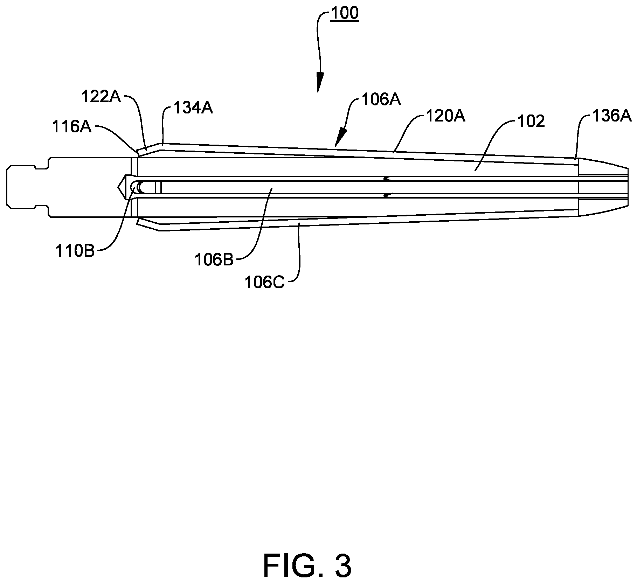

As shown in FIG. 3, fin 106A may include a main body 120A and a tip 122A that is angled with respect to main body. In further detail, tip 122A of fin 106A extends from an end of main body 120A to free end 116A of fin 106A. Tip 122A of fin 106A is oriented such that when fin 106A is in a neutral state, i.e., with no forces applied against it, free end 116A is closer to elongate body 102 than an opposite end 134A of tip at the end of main body 120A. In some examples, tip 122A of fin 106A includes a backward curve to assist in removal of the intramedullary stem from the medullary canal. In other words, tip 122A extends transverse from main body 120A of fin 106A, as shown in FIG. 3. As mentioned above, the attachment of fin to elongate body may be via a living hinge 136A. The living hinge holds the fin in a manner such that the fin is flexible. Further, in a neutral, unloaded condition, the living hinge may hold the fin such that the fin is positioned external to the outer surface of the elongate body along its length. When force is applied to the fin, the fin is compressed inwards into the lumen of the stem about the living hinge. In some examples, and as shown in FIGS. 1A-1B, first slit 110A and fin 106A are sized such that free end 116A of fin is movable into first slit. In some examples, a length of fin 106A between first slit end 112A and free end 116A is greater than half of length 104 of elongate body 102.

In some examples, such as that shown in FIGS. 1A-1B, the stem 100 may have four fins complimentary to four first slits. However, the number of fins are not limited as such and in other examples, the stem may have one or more fins and a corresponding number of first slits to receive the one or more fins. In some examples, the stem may include a plurality of first slits and a plurality of fins. The plurality of first slits may extend from a first slit end to a second slit end along the elongate body. Each first slit of the plurality of first slits may be in between the first and second ends of the elongate body. Each first slit of the plurality of first slits may include an elongate dimension aligned with the elongate body. The plurality of fins may correspond to the plurality of first slits. Additionally, each fin of the plurality of fins may be attached to a respective first slit of the plurality of first slits. In some examples, a circumferential spacing between a first pair of first slits from among the plurality of first slits may be different from a second pair of first slits from among the plurality of first slits. In other examples, the stem may have one or more corresponding first slits and fins that are not distributed uniformly across the circumference of the stem.

In some examples, at least one first slit of the plurality of first slits may be different from another slit of the plurality of first slits. Similar variations may also be included in any two fins of the plurality of fins. In other examples, a number of the first slits may be different from a number of the fins and as such at least one slit or fin may not have a complementary fin or slit. In other variations of the stem, the body may include one or more fins with corresponding first slits, while the body does not include any second slits similar to second slits. In other examples of such variations, a number of second slits may be different from a number of fins. In some examples, the stem may include one, two, three, or five or more fins and fins that have non-uniform spacing around stem body. In some examples, the first and second slits and the fins may have lengths and widths that vary from that shown in the depicted embodiment.

In some examples, the outer surface of elongate body may include spines or ridges to assist in rotational stability while allowing fins to dictate proximal and distal positioning and angling of the stem. In some examples, an outward facing surface of the elongate body may include one or more ridges. In some examples, the elongate body of stem may be curved or bowed. In other examples, a proximal portion of elongate body may be linear while a tip portion of elongate body may be curved. In some examples, the stem may have an outer surface that is parallel with a central longitudinal axis of the stem along its elongate dimension. In other examples, the outer surface of the stem may be tapered along its elongate dimension. In some examples, the intramedullary stem may be a threaded stem, e.g., with threads on an outer surface of elongate body such that the stem may be accepted by a threaded femur or tibia without the need for an adapter, such as a modular taper adapter.

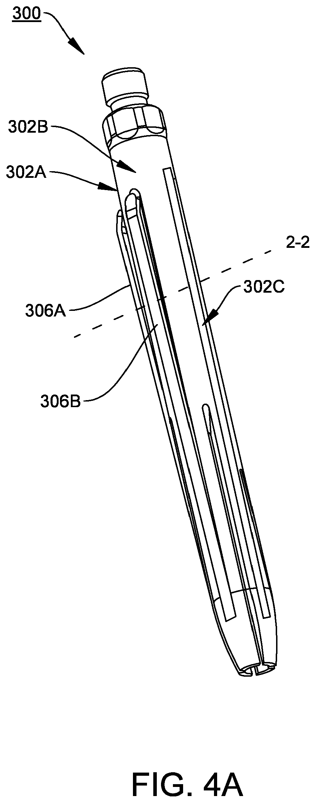

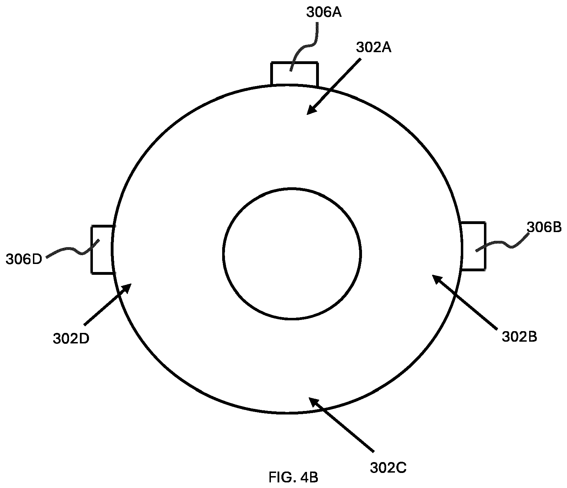

In some examples, an intramedullary stem of the present disclosure may be configured to promote engagement with bone in an asymmetric manner. Specifically, the fins may be irregularly distributed around a periphery of the elongate body. Such arrangement may induce the stem to bend in a predetermined manner when advanced into a medullary canal. In such examples, as shown in FIG. 4A, stem 300 includes one or more radial portions. When divided into quadrants, stem 300 includes a first radial portion 302A, a second radial portion 302B, a third radial portion 302C, and a fourth radial portion (not shown) each circumferentially separated and extending along a length of an elongate body of stem. One radial portion, for example, third radial portion 302C, is a solid stem portion without any fins, while each remaining radial portion, for example, first radial portion 302A, second radial portion 302B, and the fourth radial portion, includes a fin 306A, 306B where the third fin is not shown. As shown in FIG. 4B, the cross-section along line 2-2 of the elongate body of stem 300 includes first radial portion 302A having fin 306A, second radial portion 302B having fin 306B, third radial portion 302C absent a fin, and fourth radial portion 302D having fin 306D.

The fins are used to resist the driving forces coming from the distal end of the stem. In a subset of these examples, the fins are distributed around less than an entirety of the stem circumference and one or more radial portions may be absent a fin. In other examples, two or more radial portions may be a solid stem portion without any fins. Such fin placement improves the angling of the stem within the canal without the need for offsetting. Small angular changes in the direction of the stem can have large impacts on how much an implanted femoral head may overhang from a femur. In any of the above examples, a spacing between fins may vary and need not be at quarter-turn intervals around a circumference of the elongate body.

The intramedullary stem as described above may be made of a biocompatible material, such as one or more polymers and metals. Examples of polymeric materials that may be used include reinforced polymer composites, such as polyetheretherketone (PEEK), or thermoplastic polymers. Examples of metallic materials that may be used include a titanium alloy e.g., Ti6Al4V, stainless steel e.g., 316L, or other metals and metal alloys, such as Ti and CoCr.

The flexibility of each fin of the plurality of fins enables a larger tolerance band for seating a stem within a medullary canal which assists with ensuring an epiphyseal bone preparation aligns with an intramedullary stem preparation. The larger tolerance band allows for the stem to dynamically adjust to the medullary canal after insertion without reducing the fixation level. The flexible fins allow for full contact of a length of each fin with the medullary canal to ensure adequate fixation of the stem within the medullary canal, which prevents stress shielding, bone remodeling, and high load application on the bone. Further, because the stem is configured to provide adequate fixation over a range of depths and positions within the medullary canal, a user may focus on positioning of a complementary joint implant, e.g., tibial knee implant or femoral knee implant, while still obtaining adequate fixation of the stem. The fins are positioned relative to the elongate body so as to allow the fins to flex while still providing resistance along the medullary canal. In some examples, the fins may have splines or ridges to assist in rotational stability. In a subset of such examples, the fins may have one or more ridges on an outward facing surface of the fin. In some examples, the fins may be tapered. In other examples, the fins may be linear as the fins can flex slightly to adapt to the shape of the canal. The fins may be of varying widths or thicknesses.

The specific sizing of the stem is critical to the stability and longevity of the stem implant. Proper implant selection considers design, fixation, and environmental variables including patient weight, age, bone quality and size, activity level, and preoperative level of health. The intramedullary stem is available in a variety of sizes and various diameters. In some examples, the diameter of stem may range from 19 mm to 31 mm in 2mm increments. Additionally, stems are available in various lengths. In some examples, the length may be in a range from 50 mm to 250 mm. In a subset of these examples, the length of the stem may be in a range from 50 mm to 150 mm. In other examples, the length may be greater than 150 mm.

As to manufacture, the stem may be manufactured using, for example, injection molding; milling; an additive manufacturing process, such as 3D printing; lathe; or wire EDM and forming operations from conventional machining, such as bending a slit cut from a cylinder using wire EDM into a specific shape of the desired fin. Additive manufacture, when used, may be performed as described in U.S. Pat. Nos. 4,863,538, 5,017,753, 5,076,869, 4,944,817, 7,537,664, 10,614,176, 11,534,307 and 11,737,880, the entire disclosures of which are hereby incorporated by reference herein.

In another aspect, the present disclosure relates to surgical kits for implantation of a femoral implant having an intramedullary stem. A kit may include any of the various intramedullary stems described herein in any combination and in any quantity. For example, in some embodiments, two or more of the intramedullary stems may be combined in a kit. In some embodiments, a kit may include one or more intramedullary stems and one or more of a femoral implant head and a femoral body that receives the femoral head, where the femoral body includes a neck and a conical portion extending from the neck. The intramedullary stems are configured to attach to the conical portion extending from the neck. In any of the above embodiments, two or more of the intramedullary stems included in a kit may be different shapes or sizes. In any one of the above embodiments, a kit may include two or more cone bodies. Such cone bodies may be the same or may be different in shape or size.

In some embodiments, a kit as contemplated in the preceding embodiments may also include various insertion instrumentation, such as reamers, distal stem trials, inserters, impactors, bone resection instrumentation, and other instrumentation used to prepare the medullary canal of the bone and insert the intramedullary stem within the canal. The contemplated kits allow an operator to have freedom to choose an appropriately sized intramedullary stem and cone body for a patient. It is also contemplated that a kit may include any combination of system components along with one or more additional instruments used to place such an intramedullary stem in a patient. In other examples, the kits contemplated herein may be accompanied by an instruction manual on how to perform one or more of the methods of using the contents of the kit.

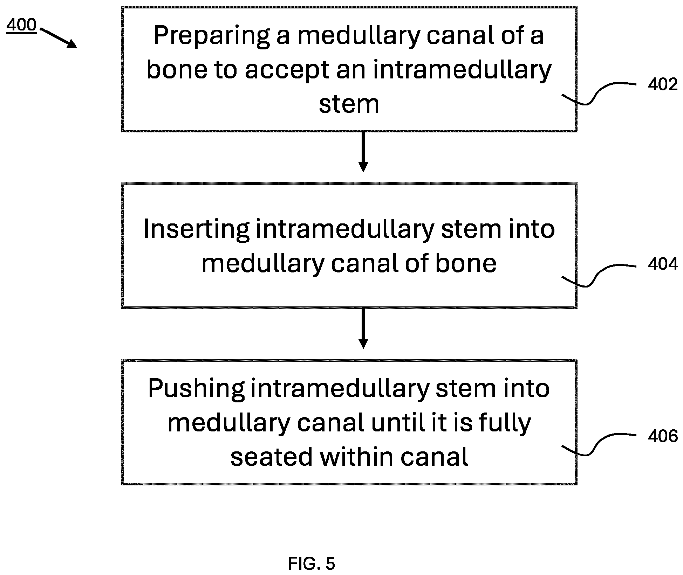

In another aspect, the present disclosure relates to a method of implanting the intramedullary stem into a long bone. Examples of the long bone that may receive the intramedullary stem include a femur and a tibia. One embodiment of the method of implantation is method 400 shown in FIG. 5. Method 400 of implanting the stem into a long bone includes a step 402 of preparing a medullary canal of a bone to accept an intramedullary stem, a step 404 of inserting the intramedullary stem into the medullary canal of the bone, and a step 406 of pushing the intramedullary stem into the medullary canal until it is fully seated within the canal.

In some examples, step 402 of preparing the medullary canal to accept an intramedullary stem may include using a reamer to prepare the medullary canal to accept the stem. For example, a wedge of the bone at the medial base of the greater trochanter may be removed in order to achieve neutral and lateral alignment of the canal using a reamer. A reamer may be used to create an access to the medullary canal to determine the orientation of the femoral axis for implantation of the stem. The reamer may be centralized in the canal before subsequent reaming is performed to form a hole shape for insertion of the stem. In some examples, the stem may be implanted after reaming by using a distal stem impactor adapter.

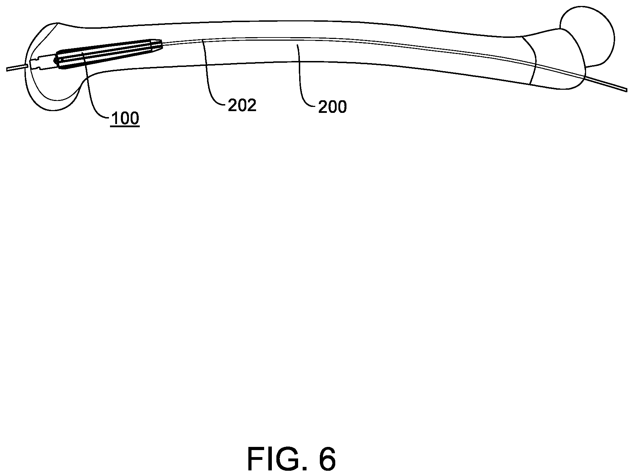

In some examples, step 404 includes inserting an intramedullary stem, such as stem 100 shown in FIGS. 1A-1B, into a medullary canal of a long bone. As shown in FIG. 6, stem 100 is inserted along a curved portion 202 of the canal 200. Stem 100 is inserted such that when stem advances into the medullary canal, at least one fin 106A from among a plurality of fins attached to an elongate body 102 of stem 100 presses against a wall surface of canal 200 and bends inward toward a slit 110A in elongate body aligned with the respective fin. Then, when stem 100 is advanced to an implantation depth within the canal, the stem is held in place and resists pull out, i.e., back out because at least one fin from among the plurality of fins is pushed inward relative to its neutral state and presses against the wall surface, thereby providing friction and resistance against movement, e.g., pull out along a length of the canal through its grip on the wall surface in the canal. In some examples, the stem may be inserted with another component attached such as a femoral component or tibial component. In some examples, the stem may include a tapered attachment. In other examples, the stem may include a threaded attachment.

In one example, step 406 of pushing the stem into the canal includes pushing the stem into the hole until it is fully seated within the canal. During the insertion step, at least one fin from among the plurality of fins is pushed toward, and in some cases into a corresponding slot along the elongate body to allow for ease of insertion. Once the intramedullary stem is at the implantation depth, the intramedullary stem may be offset from a centerline of the medullary canal while still being fixed in place within the canal. The fins, pushed inward by contact with a wall of the canal, secure the stem in place.

It should be noted that any of intramedullary stems and methods disclosed herein may be used in conjunction with robotic technology. For example, any of the stems described herein may be used with robotic surgical systems for implantation. Further, any or all of the steps described in the method of implanting the intramedullary stem in the medullary canal may be performed using a robotic system.

It is to be understood that the disclosure set forth herein includes any possible combinations of the particular features set forth above, whether specifically disclosed herein or not. For example, where a particular feature is disclosed in the context of a particular aspect, arrangement, configuration, or arrangement, that feature may also be used, to the extent possible, in combination with and/or in the context of other particular aspects, arrangements, configurations, and arrangements of the technology, and in the technology generally.

Although the disclosure herein has been described with reference to particular embodiments, it is to be understood that these embodiments are merely illustrative of the principles and applications of the present disclosure. For example, features described in relation to one particular embodiment may be combined with features of other embodiments described herein. It is therefore to be understood that numerous modifications may be made to the illustrative embodiments and that other arrangements may be devised without departing from the spirit and scope of the present disclosure as defined in the appended claims.

## Claims

1. An intramedullary stem (100, 300) for implantation in a bone, the intramedullary stem comprising:
an elongate body (102, 302A-302D) having a length (104) extending from a first end (124) to a second end (126) opposite the first end (124), the elongate body (102, 302A-302D) having a lumen (118) along at least a portion of the length (104);
a first slit (110A-110C) extending from a first slit end (112A-112C) to a second slit end (114A-114C) along the elongate body (102, 302A-302D), the first slit (110A-110C) being in between the first and second ends (124, 126) of the elongate body (102, 302A-302D) and the first slit (110A-110C) having an elongate dimension aligned with the elongate body (102, 302A-302D);
a fin (106A-106C, 302A, 302B, 302D) attached to the elongate body (102, 302A-302D) at the first slit end (112A-112C) and extending to a free end (116A-116C) remote from the first slit end, the fin (106A-106C, 302A, 302B, 302D) being biased in a first position where the free end (116A-116C) is external to an outer surface of the elongate body (102, 302A-302D) and being movable based on radially inward forces to push the free end toward the elongate body (102, 302A-302D); and
a second slit (108A-108C) extending from the first end (124) of the elongate body (102, 302A-302D) to an internal end (125A-125C) along the elongate body (102, 302A-302D), the second slit (108A-108C) having an elongate dimension aligned with the elongate body (102, 302A-302D).

2. The intramedullary stem of claim 1, wherein the first slit (110A-110C) and the fin (106A-106C) are sized such that the free end (116A-116C) of the fin (106A-106C) is movable into the first slit (110A-110C).

3. The intramedullary stem of claim 1 or claim 2, wherein a length of the fin (106A-106C) between the first slit end (110A-110C) and the free end (116A-116C) is greater than half of the length (104) of the elongate body (102).

4. The intramedullary stem of any one of claims 1-3, wherein the attachment of the fin (106A-106C) to the elongate body (102) is a living hinge (136A).

5. The intramedullary stem of any one of claims 1-4, wherein the fin (106A-106C) includes a main body (120A) and a tip (122A) that is angled with respect to the main body (120A), the tip (122A) extending from an end of the main body (120A) to the free end (116A), and the tip (122A) being oriented such that the free end (116A) is closer to the elongate body (102) than an opposite end (134A) of the tip (122A) at the end of the main body (120A).

6. The intramedullary stem of any one of claims 1-5, wherein the lumen (118) includes an inner surface, the inner surface being either parallel or tapered relative to a central longitudinal axis (119) of the elongate body (102).

7. The intramedullary stem of any one of claims 1-6, wherein the fin (106A-106C) includes one or more ridges on an outward facing surface of the fin (106A-106C).

8. The intramedullary stem of any one of claims 1-7, wherein an outward facing surface of the elongate body (102) includes one or more ridges.

9. The intramedullary stem of any one of claims 1-8, wherein the elongate body (102) is curved.

10. The intramedullary stem of any one of claims 1-9, wherein the fin (106A-106C). is a first fin, and the stem further comprises:
a plurality of slits (110A-110C) each extending from a first slit end (112A-112C) to a second slit end (114A-114C) along the elongate body (102), each slit of the plurality of slits (110A-110C) being in between the first and second ends (124, 126) of the elongate body (102) and each slit of the plurality of slits (110A-110C) having an elongate dimension aligned with the elongate body (102); and
a plurality of fins (106A-106C) corresponding to the plurality of slits (110A-110C), each fin of the plurality of fins (106A-106C) being attached to a respective slit of the plurality of slits (110A-110C),
wherein a circumferential spacing between a first pair of slits from among the first slit and the plurality of slits is different from a second pair of slits from among the first slit and the plurality of slits, and
wherein at least one slit of the first pair of slits is different from the second pair of slits.
